# FASCICULE DE BREVET EUROPEEN

(11) **EP 0 334 848 B1**
(45) Date de publication et mention de la délivrance du brevet: **09.03.1994**
(21) Numéro de dépôt: 87907003.5
(22) Date de dépôt: 14.10.1987
(51) Int. Cl.: A61B 17/00, F16G 11/08

(54) **DISPOSITIF D'ABOUTAGE DE DEUX CABLES EN PARTICULIER DE TIRE-VEINE**
KUPPLUNGSVORRICHTUNG ZWEIER KABEL, INSBESONDERE FÜR VENENSTRIPPER
DEVICE FOR BENDING TWO CABLES, PARTICULARLY OF A VEIN STRIPPER

(43) Date de publication de la demande: 04.10.1989
(73) Titulaire: RUFFIE, Patrick, 33000 Bordeaux (FR)
(72) Inventeur: RUFFIE, Patrick, 33000 Bordeaux (FR)
(74) Mandataire: Thébault, Jean-Louis
(86) Numéro de dépôt international: FR8700400
(87) Numéro de publication internationale: WO8903196

## Description

La présente invention a trait à un dispositif permettant d'abouter deux câbles munis d'embout et plus particulièrement des câbles de tire-veine.

Le tire-veine ou stripper est un instrument constitué, d'un câble dont une extrémité est munie d'une fine olive sertie, d'un calibre assez petit pour permettre son cathétérisme dans la veine à extraire, en particulier la saphène interne dans le cas de varices des membres inférieurs, et dont l'autre extrémité est munie d'un pas de vis sur lequel sera fixée la boule à gorge qui permettra d'extraire la veine.

L'extraction, par exemple de la saphène interne, s'opère généralement de bas en haut ou de haut en bas, mais lorsqu'il est impossible d'effectuer cette opération en une seule fois, entre la malléole et la crosse de la saphène interne, en particulier lorsqu'il est impossible de cathétériser le tronc veineux de haut en bas, il est nécessaire de faire une incision supplémentaire à la jarretière pour procéder à l'extraction en deux fois, de l'aine à mi-cuisse ou au jarret, puis du jarret à la malléole.

Le but de l'invention est de permettre de drainer en une seule fois dans un espace clos, de manière à diminuer la fréquence des hématomes post-opératoires, en proposant des moyens permettant d'abouter, au droit d'une incision à la jarretière, deux câbles de tire-veine cathétérisés respectivement à partir des extrémités du tronc veineux, en sorte de réaliser un tire-veine unique que l'on utilisera à la manière habituelle.

On connaît déjà divers dispositifs d'aboutage de câbles ou analogues. Par exemple, le document FR - A - 2 176 322 décrit un dispositif d'aboutage comprenant deux demi-olives assemblables par vissage et comportant chacune un logement coaxial de réception de l'extrémité d'un câble ou analogue, engagée par un trou de passage ménagé axialement à la demi-olive.

Un tel dispositif n'est cependant absolument pas utilisable pour l'aboutage de câbles munis d'embouts de diamètre supérieur à celui des câbles, tels que les câbles de tire-veine.

La présente invention a pour objet un ensemble d'extraction de veine comportant deux câbles à embout de tire-veine et un dispositif d'aboutage de ces câbles constitué de deux demi-olives munies de moyens de solidarisation bout à bout et comportant chacune un logement, coaxial au corps de la demi-olive, débouchant sur les faces d'extrémité en regard des demi-olives, destiné à la réception et à la retenue dudit embout de câble et prolongé par un trou axial de passage du câble, ainsi qu'une fente latérale courant sur toute la longueur de la demi-olive et débouchant dans le logement de réception de l'embout, ladite fente étant de largeur supérieure au diamètre dudit trou axial.

Suivant un mode de réalisation, ladite fente latérale est de largeur inférieure au diamètre de l'embout et les demi-olives sont assemblées par vissage et séparables.

Suivant un autre mode de réalisation, les deux demi-olives sont assemblées de manière rotative axialement et mobiles entre deux positions, l'une, pour laquelle elles présentent en alignement deux fentes d'insertion des embouts de câble, communiquant à leurs extrémités avec lesdits logements des embouts et avec des fentes latérales de passage des câbles, de largeur inférieure au diamètre des embouts et, l'autre, pour laquelle lesdites fentes d'insertion ne sont plus en regard, empêchant ainsi toute sortie des embouts.

Avantageusement, les deux demi-olives sont assemblées par vissage permettant le verrouillage mutuel desdites demi-olives dans ladite autre position.

D'autres caractéristiques et avantages ressortiront de la description qui va suivre de modes de réalisation du dispositif de l'invention, description donnée à titre d'exemple uniquement et en regard du dessin annexé sur lequel:
- Figure 1 représente les extrémités de deux câbles à embout de tire-veine à abouter ;
- Figure 2 représente une vue en perspective d'un premier mode de réalisation du dispositif de l'invention ;
- Figure 3 représente une vue en coupe axiale des deux demi-olives séparées du dispositif de la figure 2 ;
- Figure 4 est une vue de dessus d'un second mode de réalisation du dispositif de l'invention, et
- Figure 5 est une coupe axiale du dispositif de la figure 4.

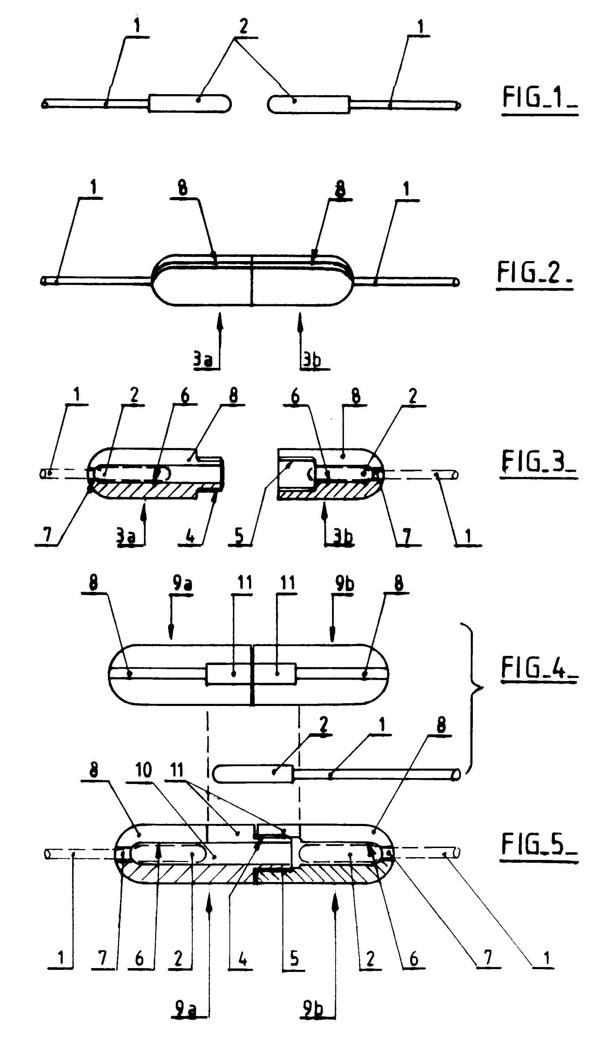

La figure 1 illustre deux câbles 1 munis à leur extrémité d'embout 2 cylindrique de diamètre légèrement supérieur à celui du câble. Les câbles 1 et embout 2 sont par exemple en matière plastique et du type utilisé pour constituer des tire-veines.

La figure 2 illustre un premier mode de réalisation d'un dispositif pour relier bout à bout les embouts 2 de deux câbles 1 en vue d'en faire un câble unique. Il s'agit d'un mode de réalisation simplifié constitué de deux demi-olives 3a,3b symétriques, excepté en ce qui concerne les moyens de solidarisation des demi-olives, formés par un prolongement tubulaire fileté 4, coaxial à l'une (3a) des demi-olives et par un trou taraudé 5 de réception, coaxial à l'autre demi-olive (3b).

Les demi-olives 3a,3b, qui sont en métal ou une matière plastique appropriée à l'usage auquel il est destiné, ont une forme générale cylindrique arrondie à leur extrémité opposée à leur face de raccordement mutuel. Le diamètre des demi-olives correspond à celui des olives habituellement utilisées dans les tire-veines.

Chaque demi-olive 3a,3b comporte un logement cylindrique coaxial 6 de réception d'un embout 2, débouchant sur l'extrémité arrondie de la demi-olive par un trou 7 de diamètre supérieur à celui des câbles 1 mais inférieur à celui des embouts 2.

Dans la demi-olive 3a, le logement 6 traverse le prolongement fileté 4 et débouche sur l'autre extrémité de la demi-olive. Dans la demi-olive 3b, le logement 6 débouche dans le trou taraudé 5, lequel est bien entendu de diamètre supérieur à celui des logements 6. La longueur des demi-olives 3a,3b est au moins légèrement supérieure à celle des embouts 2.

En outre, les logements 6 sont munis d'une fente latérale d'accès 8 courant sur toute la longueur des demi-olives depuis leur faces d'accouplement jusqu'aux trous 7 de passage des câbles 1. La largeur des fentes 8 est supérieure au diamètre des câbles 1, mais inférieure à celui des embouts 2.

Pour mettre en place les embouts 2 dans les logements 6, il faut dévisser les demi-olives 3a,3b et les séparer, engager dans chacune l'un des câbles 1 par la fente latérale 8 et tirer sur le câble pour faire pénétrer l'embout dans son logement 6. Les embouts 2 étant alors dans la position représentée sur la figure 3, les demi-olives 3a,3b sont revissées à fond de façon à obtenir l'assemblage illustré par la figure 2, les fentes 8 se trouvant (ceci n'étant d'ailleurs pas indispensable) dans le prolongement l'une de l'autre.

Les deux câbles 1 sont ainsi parfaitement aboutés et ne font qu'un.

Une telle opération d'aboutage s'effectue au droit de l'incision à la jarretière, lorsque les deux extrémités de câbles 1 sont parvenues par cathétérisation à cette incision. On aboute alors les deux extrémités avec le dispositif de l'invention qui est ensuite inséré dans le tronc veineux pour permettre le "strippage" à la manière habituelle de l'ensemble du tronc entre la malléole et la crosse de la saphère interne, par le haut ou par le bas.

Les figures 4 et 5 illustrent un mode de réalisation préféré dans lequel il n'est pas nécessaire de séparer les deux demi-olives pour mettre en place ou enlever les embouts 2.

Dans ce mode de réalisation, les demi-olives 9a,9b ont la même forme générale que dans le mode de réalisation précédent, c'est à dire cylindrique à bout arrondi percé d'un trou 7 de passage du câble 1.

La solidarisation des demi-olives s'effectue de la même manière par un prolongement fileté 4 engagé dans un trou taraudé 5.

Chaque demi-olive 9a,9b comporte également un logement cylindrique axial 6 de réception d'un embout 2, mais la longueur des demi-olives est accentuée de façon à être au moins égale à un peu plus de trois fois la longueur d'un embout. Ceci permet de ménager dans la partie médiane de l'assemblage 9a,9b, à cheval sur les deux demi-olives, un logement cylindrique 10 de réception d'un embout 2, situé dans le prolongement des logements 6 et communiquant avec eux.

Les logements 6 sont également munis d'une fente latérale 8 de passage de câble, de largeur inférieure au diamètre des embouts 2, cependant qu'au droit du logement central 10 les fentes 8 se prolongent par une fente 11 de largeur accrue correspondant au diamètre des embouts 2 et de longueur légèrement supérieure à celle de ces derniers.

Pour l'insertion des embouts 2 dans le dispositif, les demi-olives 9a,9b sont amenées par rotation mutuelle dans la position de la figure 4 pour laquelle les fentes 8,11 sont alignées. L'un des embouts 2 est inséré dans le logement en deux parties 11, le câble 1 étant engagé dans la fente 8 adjacente. Le câble est alors tiré pour amener l'embout dans le logement 6 de l'une des demi-olives.

On effectue la même chose pour l'autre embout qu'on insère dans l'autre demi-olive, comme illustré par la figure 5.

Il suffit enfin de décaler angulairement l'une des demi-olives par rapport à l'autre, de préférence en poursuivant la rotation jusqu'au verrouillage mutuel des demi-olives, de manière que les demi-logements 11 ne soient plus en regard, interdisant ainsi toute extraction d'un embout. Il n'y a alors aucun risque de dévissage des demi-olives, ni de décrochage accidentel de l'un des embouts en cours d'utilisation du dispositif selon l'invention.

L'enlèvement des embouts 2 s'effectue par une manipulation strictement inverse.

Enfin, l'invention n'est évidemment pas limitée aux modes de réalisation représentés et décrits ci-dessus mais en couvre au contraire toutes les variantes notamment en ce qui concerne la nature des moyens de solidarisation des demi-olives, ainsi que les formes et dimensions des logements de réception-retenue des embouts des câbles à abouter.

## Revendications

1. Ensemble d'extraction de veine comportant deux câbles (1) à embout (2) de tire-veine et un dispositif d'aboutage de ces câbles constitué de deux demi-olives (3a,3b ; 9a,9b) munies de moyens de solidarisation bout à bout (4,5) et comportant chacune un logement (6), coaxial au corps de la demi-olive, débouchant sur les faces d'extrémité en regard des demi-olives, destiné à la réception et à la retenue dudit embout (2) de câble et prolongé par un trou axial (7) de passage du câble (1), ainsi qu'une fente latérale (8) courant sur toute la longueur de la demi-olive et débouchant dans le logement (6) de réception de l'embout (2), ladite fente (8) étant de largeur supérieure au diamètre dudit trou axial (7).

2. Ensemble d'extraction de veine selon la revendication 1, caractérisé en ce que ladite fente latérale (8) est de largeur inférieure au diamètre de l'embout (2) et en ce que les demi-olives (3a,3b) sont assemblées par vissage et séparables.

3. Ensemble d'extraction de veine selon la revendication 1, caractérisé en ce que les deux demi-olives (9a,9b) sont assemblées de manière rotative axialement et mobiles entre deux positions, l'une, pour laquelle elles présentent en alignement deux fentes (11) d'insertion des embouts (2) de câble, communiquant à leurs extrémités avec lesdits logements (6) des embouts (2) et avec des fentes latérales (8) de passage des câbles (1), de largeur inférieure au diamètre des embouts (2) et, l'autre, pour laquelle lesdites fentes d'insertion (11) ne sont plus en regard, empêchant ainsi toute sortie des embouts.

4. Ensemble d'extraction de veine selon la revendication 3, caractérisé en ce que les deux demi-olives (9a,9b) sont assemblées par vissage permettant le verrouillage mutuel desdites demi-olives dans ladite autre position.

## Claims

1. Vein-extraction assembly comprising two vein stripper cables (1) with end pieces (2) and a device for joining these cables consisting of two half-olives (3a, 3b; 9a, 9b) provided with means of connecting end to end (4, 5) and each having a recess (6) coaxial with the body of the half-olive, opening out on the opposite end faces of the half-olive, intended to receive and hold the said cable end piece (2) and extended by an axial hole (7) for the cable (1) to pass through, as well as a lateral slot (8) running along the entire length of the half-olive and opening out in the recess (6) for receiving the end piece (2), the said slot (8) having a width greater than the diameter of the said axial hole (7).

2. Vein-extraction assembly according to claim 1, characterised in that the said lateral slot (8) has a width less than the diameter of the end piece (2 ) and in that the half-olives (3a, 3b) are connected by screwing and can be separated.

3. Vein-extraction assembly according to claim 1, characterised in that the two half-olives (9a, 9b) are connected in an axially rotating manner and can move between two positions, one in which they have two slots (11) in alignment for inserting the cable end pieces (2), communicating at their ends with the said recesses (6) in the end pieces (2) and with lateral slots (8) for the cables (1) to pass through, of a width less than the diameter of the end pieces (2), and the other in which the said insertion slots (11) are no longer opposite each other, thus preventing the end pieces from coming out.

4. Vein-extraction assembly according to claim 3, characterised in that the two half-olives (9a, 9b) are connected by screwing, enabling the said half-olives to be mutually locked in the said other position.

## Patentansprüche

1. Venenextraktionsbaugruppe umfassend zwei Venenziehkabel (1) mit Kabelmarke (2) und eine Vorrichtung zum Verbinden dieser Kabel auf Stoß, bestehend aus zwei Halboliven (3a,3b;9a,9b), die mit Mitteln zum Verbinden auf Stoß (4, 5) versehen sind und jeweils eine Aufnahme (6) koaxial zum Korpus der Halbolive umfassen, welche auf den Endflächen gegenüber den Halboliven münden, bestimmt zur Aufnahme und Halterung der Kabelmarke (2) des Kabels und verlängert um ein axiales Loch (7) für den Durchtritt des Kabels (1), wie auch mit einem seitlichen Spalt (8) versehen, der über die gesamte Länge der Halbolive läuft und in der Ausnehmung (6) für die Aufnahme der Kabelmarke (2) mündet, welcher Spalt (8) eine Breite aufweist, die größer ist als der Durchmesser des axialen Loches (7).

2. Venenextraktionsbaugruppe nach Anspruch 1, dadurch gekennzeichnet, daß der seitliche Spalt (8) eine Breite aufweist, die kleiner ist als der Durchmesser der Kabelmarke (2) und daß die Halboliven (3a, 3b) durch Verschrauben gefügt sind und lösbar sind.

3. Venenextraktionsbaugruppe nach Anspruch 1, dadurch gekennzeichnet, daß die beiden Halboliven (9a, 9b) durch Drehung axial gefügt sind und zwischen zwei Positionen beweglich sind, einer, in der sie zwei Spalte (11) in Ausfluchtung für das Einfügen der Kabelmarke (2) des Kabels aufweisen, in Kommunikation an ihren Enden mit den besagten Aufnahmen (6) die Kabelmarke (2) und mit den seitlichen Spalten (8) für den Durchtritt der Kabel (1) mit einer Breite, die kleiner ist als der Durchmesser die Kabelmarke (2) und, der anderen, in der die Einfügespalten (11) sich nicht mehr gegenüberstehen, wodurch jedes Heraustreten die Kabelmarke gesperrt wird.

4. Venenex traktionsbaugruppe nach Anspruch 3, dadurch gekennzeichnet, daß die beiden Halboliven (9a,9b) durch Verschrauben gefügt sind, was die gegenseitige Verriegelung der Halboliven in der genannten zweiten Position ermöglicht.
